# EUROPEAN PATENT APPLICATION

(11) **EP 3 450 564 A1**
(43) Date of publication of application: **06.03.2019**
(21) Application number: 17189513.9
(22) Date of filing: 05.09.2017
(51) Int. Cl.: C12P 7/06, C12M 1/107, C12P 7/16, C12P 7/28

(54) **METHOD OF PRODUCING ORGANIC SOLVENTS IN A BIOREACTOR**

(71) Applicant: VITO NV, 2400 Mol (BE)
(72) Inventor: VAN HECKE, Wouter, B-2400 Mol (BE); DE WEVER, Helene, B-2400 Mol (BE); BECKERS, Herman, B-2400 Mol (BE)
(74) Representative: Pronovem

(57) **Abstract**

Method of producing an organic solvent, comprising adding a feed (14) to a medium (138) comprised in a reactor (13), converting the feed in the reactor to form the organic solvent, withdrawing a first stream (15) from the medium through a first membrane (115), and withdrawing a second stream (16) from the medium through a second membrane (125), the second stream comprising the organic solvent. The first membrane and the second membrane are moved relative to the reactor during the steps of withdrawing the first stream and withdrawing the second stream.

## Description

### Technical field

The present invention is related to methods for producing organic solvents through fermentative processes, in particular for producing solvents such as butanol or acetone.

### Background art

Early in the 20th century, the microorganism *Clostridium acetobutylicum* was found to convert carbohydrate containing feedstocks into acetone, ethanol and n-butanol, as described in U.S. Patent Nos. 1,315,585, and 2,386,374. The method has been referred to since as the acetone n-butanol ethanol (ABE) fermentation process.

As generally known, and for example described in WO2013/086458 and WO2015/002913, n-butanol is an important industrial chemical, useful for example as a solvent, as a feedstock chemical in the plastics industry, as a fuel additive, as an ingredient in formulated products such as cosmetics, and as a food grade extractant in the food and flavor industry. Moreover, as a fuel, n-butanol has several advantages over ethanol. For instance, while n-butanol can be made from the same feedstocks as ethanol, it is, unlike ethanol, compatible with gasoline and diesel at higher ratios. Furthermore, n-butanol can also be used alone as a pure fuel in existing cars without modifications, it has been proposed as a building block to make jet fuel, etc.

A major drawback of n-butanol, however, is its toxicity to the producing culture in the ABE fermentation process, leading to cell inhibition. This is for example discussed in WO2013/086458 and EP 2283141. Because of such end product toxicity, solvent productivity is limited and the final concentration of product on a volume basis is low as well. Consequently, energy-intensive distillation operations are used, negatively affecting the economics of recovery of the different products. The high purification cost was one of the major reasons why the ABE fermentation was to a large extent abandoned during the 1950s and 1960s and replaced by petroleum based chemical plants for production of n-butanol and acetone. As such, each year 10 to 12 billion pounds of n-butanol are produced by petrochemical means. However, the depletion of today's fossil fuel stocks, the fluctuations in fossil fuel price and security of energy sources are the driving forces behind the current revival in n-biobutanol production. Accordingly, there is a high demand for efficient and sustainable methods for the production of n-butanol.

As nowadays there is an interest in development of technologies that use renewable resources for fuel production, the ABE fermentation is attracting renewed interest. However, solutions have to be found to avoid or reduce the n-butanol toxicity leading to cell inhibition and the associated low productivities and high purification costs.

In the art, it has already been proposed to alleviate the product inhibition by complementing the fermentation process with in situ product recovery (ISPR) technologies, such as adsorption, pervaporation, gas stripping, or liquid/liquid extraction. In this way, n-butanol is removed from the fermentor as it is produced, thereby allowing the microorganism to produce n-butanol at higher productivity.

WO 2011/160030 for example utilizes liquid-liquid extraction as ISPR in a method and system for efficiently producing a fermentative product alcohol such as n-butanol.

Despite these efforts, the above methods still remain quite energy-intensive, in particular because they require performing pumps which provide for sufficient cross-flow streams on the membranes to avoid fouling and concentration and temperature gradients. Furthermore, substantial peripheral components are required around the reactor in order to obtain the above efficiency improvements.

### Summary of the invention

It is therefore an aim to provide methods which overcome the above drawbacks. In particular, it is an aim to provide methods which are more efficient in producing organic solvents from substantially aqueous feeds. It is furthermore an aim to provide methods which have a reduced complexity and/or allow for simplifying downstream processing.

According to a first aspect of the invention, there is therefore provided a method of producing an organic solvent as set out in the appended claims. A feed is added to a medium comprised in a reactor. The feed is converted in the reactor to form the organic solvent. A first stream is separated or withdrawn from the medium through a first membrane, which is advantageously provided in the reactor, e.g. is advantageously (at least partially) immersed in the medium. The first membrane is advantageously a semipermeable membrane. A second stream is withdrawn from the medium through a second membrane. The second stream is separated or withdrawn advantageously in parallel (e.g., simultaneously) with withdrawing the first stream. The second membrane is advantageously housed in the reactor and is advantageously (at least partially) immersed in the medium. The second membrane is advantageously a semipermeable membrane. The second stream advantageously comprises the organic solvent. In addition, the first stream may comprise the organic solvent.

According to an aspect, the first membrane and the second membrane are moved relative to the reactor, advantageously during the steps of withdrawing the first stream and withdrawing the second stream.

Apparatuses for carrying out the methods as described are described herein as well.

By moving the membranes relative to the reactor, less pumping energy is required for sustaining a desired cross flow over the membrane surface. Furthermore, by appropriately maintaining the reactor medium in motion relative to the membranes simplifies the requirements for feed supply control, such as for temperature and concentration gradient control. Temperature gradients are minimized, and concentration gradients over a surface of the membranes can easily be minimized. A further advantage of the apparatuses and processes described herein is that they are inherently safer because no associated (external) loops with high cross flows are involved. There is no or less external piping which may be prone to leakage and/or which may expose maintenance operators to (potentially) toxic products. A further advantage is that they allow for integrating the membranes within the reactor vessel resulting in reactors with small footprints, taking much less space in comparison to prior art designs.

### Brief description of the figures

Aspects of the invention will now be described in more detail with reference to the appended drawings, wherein same reference numerals illustrate same features and wherein:
Figure 1A represents schematically an apparatus integrating two separate membrane units in the reactor according to aspects described herein;
Figure 1B represents schematically an alternative of the apparatus of Fig. 1A, in which a third membrane unit is further provided in the reactor;
Figures 2A-B represent a horizontal respectively vertical cross section of a reactor according to aspects described herein, wherein two membrane units are mounted on rotating supports within the reactor vessel; Fig. 2B represents a cross section view along section line A-A;
Figure 3 represents a (horizontal) cross section of an alternative reactor compared to Fig. 2A, wherein the two membrane units are mounted on a single rotating support within the reactor vessel;
Figure 4 represents a downstream process block diagram for the separation and purification of the streams recovered from the fermentation reactors of the apparatus of Figs. 1A and 1B.

### Description of embodiments

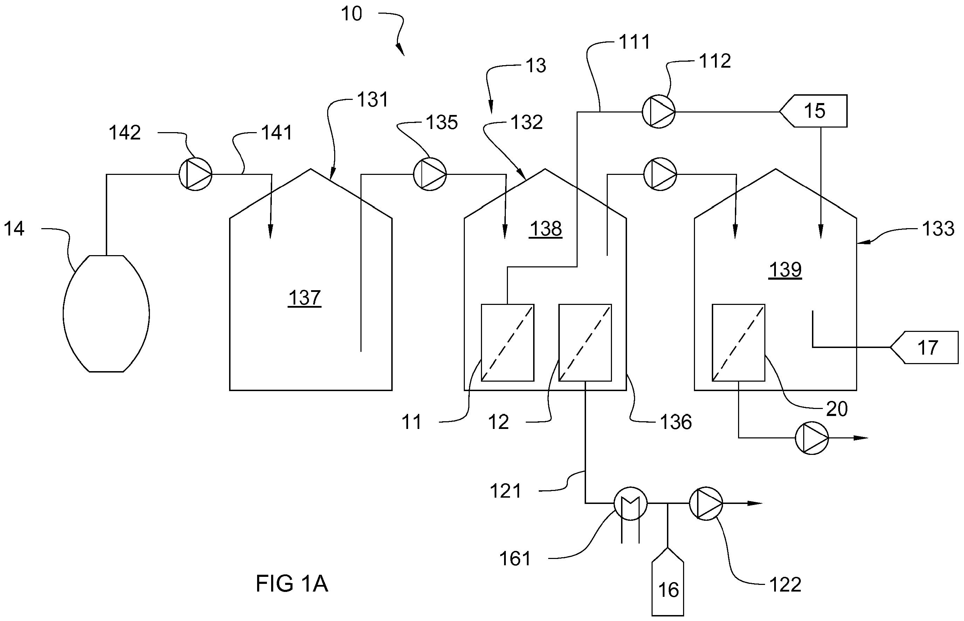

Referring to Fig. 1A, an apparatus 10 according to aspects described herein integrates two separate, distinct membrane units 11 and 12 within a reactor 13. Reactor 13 is advantageously a reactor for conversion of a feedstock 14, in particular a biomass feedstock, to a useful product, in particular an organic solvent, or a mixture of a plurality of organic solvents. The conversion may be assisted by a suitable (bio)catalyst, such as a microorganism and/or an enzyme. Feed 14 is advantageously continuously supplied to the reactor 13 through a supply duct 141 via a feed pump 142.

The conversion reaction(s), in aspects of the present description, advantageously refer to fermentation reactions, including fermenting a feedstock in the presence of microorganisms, such as microorganisms of the Clostridium genus, in particular *Clostridium acetobutylicum.* The fermentation reaction(s) are advantageously all or in part carried out under anaerobic conditions.

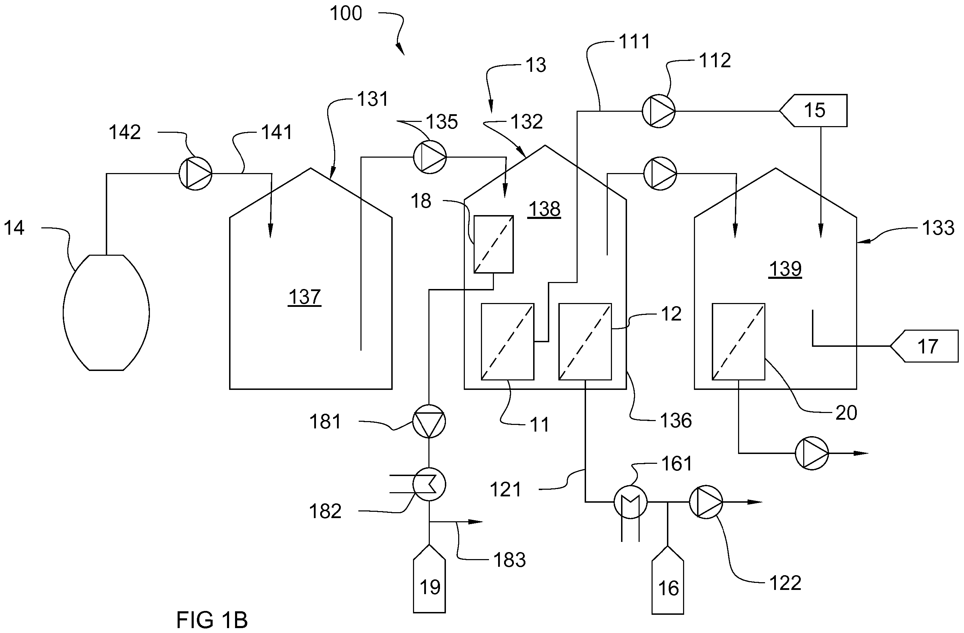

Reactor 13 may be a single stage reactor, e.g. comprising a single vessel in which the feedstock 14 is converted; or a multi-stage reactor, comprising a plurality of reactor vessels arranged in series or in cascade, in which the conversion/fermentation reactions are carried out in a stepwise manner. By way of example, the reactor can be a two-stage reactor, or a three-stage reactor, as shown in Fig. 1. The three-stage reactor 13 comprises a first reactor 131 serially coupled to a second reactor 132, which in turn is serially coupled to a third reactor 133. The first reactor 131 comprises a first medium 137, which may comprise partially converted feedstock 14 and may also comprise residual feedstock that is not converted in the first reactor 131. Medium 137 is advantageously continuously withdrawn from the first reactor and supplied to the second reactor 132, e.g. via a pump 135. The second reactor 132 comprises a second medium 138, in which the medium 137 of the first reactor, which may contain residual feedstock 14, is further reacted. The second medium 138 may already comprise desired reaction products, such as a mixture of ABE solvents. The second medium 138 is advantageously continuously withdrawn from the second reactor and supplied to the third reactor 133, in which residual feedstock, such as residual carbohydrates, can further be converted in reactor medium 139. A third stream 17 may be withdrawn from the third reactor 133. It will be convenient to note that any one or all of the media 137, 138, 139 may be referred to as a fermentation broth.

Microorganisms may be present in any one or all of the first reactor 131, the second reactor 132, and the third reactor 133. By way of example, in the first reactor 131, an acidogenic fermentation may take place. In the second and third reactors 132 and 133, a solventogenic fermentation may take place. The third reactor may allow for maximizing solvent titers. The improved carbohydrate conversion will lower the substrate costs and the higher solvent titers will decrease the cost for further recovery of residual solvents. It will be convenient to note that more reactor stages may be added as desired, or any one of the first reactor stage and the third reactor stage may be omitted.

The feedstock 14 may comprise any suitable biomass. Feedstock 14 may originate from or comprise sugar cane, corn mash, or wheat. The feedstock 14 advantageously comprises carbohydrates, in particular hydrolysates, such as C5/C6 carbohydrates (such as starch, glucose, xylose), lignocellulosic hydrolysates, or hydrolysates from pulp and paper industry.

Advantageously, the fermentation reaction(s) in aspects of the present invention is (are) carried out at a temperature comprised between 30°C and 45°C, advantageously between 30°C and 40°C, advantageously between 32°C and 38°C, advantageously between 35°C and 37°C. This is obtained by maintaining the medium in reactor 13 (e.g. either one of the first reactor 131, second reactor 132, third reactor 133, or any combination thereof), at the temperature as indicated.

Advantageously, the pH of the medium in reactor 13 is maintained between 4.0 and 6.0, advantageously between 4.0 and 5.5, advantageously between 4.5 and 5.5, advantageously between 4.5 and 5.0.

Methods in aspects of the present description can be performed in a batch, fed-batch, or continuous manner, i.e. the feedstock 14 is provided (or introduced) in the reactor 13 on a batch, fed-batch, or continuous basis.

The microorganisms are advantageously housed in the reactor 13, and may or may not be immobilised therein. Reaction products and possibly additional compounds, are advantageously continuously removed from the reactor 13 through membrane units 11 and 12. Either one of, or both the membrane units 11 and 12 is advantageously configured to recover or withdraw specific compounds. The membrane units 11 and 12 advantageously allow for retaining the microorganisms in the reactor.

A first membrane unit 11 may be configured to withdraw a first stream 15 from reactor 13. Membrane unit 11 is advantageously configured as a filtration unit, comprising filtration membranes, for producing the first stream 15 as a permeate stream from the medium in the reactor 13. Membrane unit 11 is coupled to an outlet duct 111 at the permeate side. A pump 112 in communication with the outlet duct 111 may be configured for installing a desired pressure difference across the membranes of membrane unit 11.

The first stream 15 is advantageously withdrawn in order to increase cell concentration (of dry cell weights) in the reactor. This may advantageously result in an increase of feed (substrate) supply rates, solvent productivity and/or utilization of xylose. Additionally, the filtration membranes of the first membrane unit advantageously allow for retaining microorganisms and other possible catalysts in the reactor medium. This allows for increasing cell/microorganism concentration in the reactor, which may improve product conversion and may allow for reducing residence times of the feedstock in the reactor.

The membranes of first membrane unit 11 are advantageously semipermeable membranes, advantageously filtration membranes. Specific examples of suitable filtration membranes are microfiltration and ultrafiltration membranes. Another useful example is nanofiltration membranes.

A second membrane unit 12 may be configured to withdraw a second stream 16 from reactor 13. The second membrane unit 12 is advantageously configured for withdrawing the second stream 16 through pervaporation (or liquid-gas extraction) or liquid-liquid extraction. To this end, the second membrane unit 12 may comprise one or more semipermeable membranes. In a first alternative, the second membrane unit 12 may be provided as a pervaporation unit, comprising semipermeable membranes which are advantageously suitable for use in pervaporation processes. Alternatively, the second membrane unit 12 may be provided as a membrane contactor unit, comprising contactor membranes suitable for liquid-liquid extraction.

A pervaporation unit separates the second stream 16 from the medium in the reactor through permeation and evaporation of the second stream 16. In liquid-liquid extraction, a contactor membrane is used, over which a liquid phase is made to circulate at the permeate side. A difference in solubility or a concentration gradient provides the driving force to generate a mass transfer of selective compounds across the membranes, hence forming the second stream 16. The second membrane unit 12 is coupled to an outlet duct 121 at the permeate side of the unit. A pump 122, e.g. a vacuum pump in case of a pervaporation unit, may communicate with the outlet duct 121 side of the second membrane unit 12 to maintain a desired (partial vacuum) pressure level, or circulating flow (in case of liquid-liquid extraction).

The second stream 16 may comprise or consist of a reaction product, such as the organic solvent, or the mixture of organic solvents. In the particular case of ABE fermentation, e.g. with Clostridia strains, the second stream may comprise one or more of, or consist of a mixture of: isopropanol, acetone, n-butanol and ethanol, and possibly water.

In the particular case that the second membrane unit 12 is a pervaporation unit, carbon dioxide (CO₂) generated by the fermentation reactions may advantageously be collected in the second stream. Due to the generally lower pressure at the filtrate side of the filtration membranes of the first membrane unit 11, CO₂ is locally oversaturated in the filtrate side and leads to CO₂ bubble formation in the filtrate side. This distorts liquid flows and leads to a difficult control of the residence time in continuous fermentations, which is required to ensure target productivities. The pervaporation membranes for solvent recovery operated in parallel with the filtration membranes do not only decrease the solvent concentration in the fermentation broths, they also remove CO₂. This results in an improved control of the residence time in the fermentation reactors by avoiding CO₂ bubble formation at the filtrate side of the filtration membranes.

Referring to Fig. 1B, an alternative apparatus 100 and related process scheme is provided. The apparatus and process scheme of Fig. 1B differs from the one of Fig. 1A in that a third membrane unit 18 is integrated in a similar way in the reactor 13, advantageously in the same reactor vessel 136 as one or both of the other two membrane units 11 and 12. The third membrane unit 18 is configured to remove CO₂, H₂ and optionally other non-condensable gases. The third membrane unit 18 may be configured to operate as a membrane contactor, advantageously operating at a partial vacuum pressure at the permeate side. Suitable partial vacuum permeate pressures range between 75 mbar and 500 mbar, advantageously between 100 mbar and 400 mbar. At these relatively high permeate pressures (as compared to standard vacuum conditions), a negligible transfer of solvents occurs. Any solvents that may be transported through the membranes of the third membrane unit 18 may be condensed in a condenser 182 arranged downstream of a vacuum pump 181. The stream of solvents withdrawn through the third membrane unit 18 may be collected as a fourth stream 19 following removal of the non-condensable gases as a separate stream 183. Such a third membrane unit 18 can decrease overall vacuum costs. The presence of non-condensable gases greatly affects the operating costs of a vacuum pump, as shown in Van Hecke, W. and De Wever, H. in J. Membr. Sci. (2017), 540 (321-332). Hence, removal of the non-condensable gases in a separate loop at higher permeate pressures avoids excessive costs related to the vacuum (operating costs and number of vacuum pumps) in the second membrane unit 12.

According to an aspect, the membranes of the first membrane unit 11 and the membranes of the second membrane unit 12 are arranged inside the reactor 13, advantageously at least partially immersed in a medium 138 of the reactor. In particular, these membranes are arranged inside a vessel of the reactor. The reactor may be any one, or a plurality of the first reactor 131, the second reactor 132, and the third reactor 133. In the example of Fig. 1, the membranes of the first and second membrane units are arranged in the vessel 136 of the second reactor 132, advantageously at least partially and advantageously fully immersed in the second reactor medium 138. In an alternative configuration, the membranes of one or both the first and second membrane units may be arranged in the vessel of the first reactor 131, and advantageously be immersed in the first reactor medium 137. By way of example, the membranes of the first membrane unit 11 may be arranged in the first reactor 131, while the first membrane unit is arranged in the second reactor 132. Yet alternatively, an additional membrane unit operating similarly to the first membrane unit 11 may be arranged in the first reactor 131

A pervaporation or liquid-liquid extraction unit integrated within the reactor vessel simplifies the requirements for temperature and concentration gradient control. Temperature gradients are minimized, and concentration gradients over a surface of the membranes can easily be minimized by appropriately maintaining the reactor medium 132 in motion relative to the second membrane unit 12. A same reasoning applies to a membrane filtration unit, such as the first membrane unit 11.

A fifth stream can be withdrawn from the medium as a bleed. The fifth stream may be smaller in comparison to the first stream and/or the second stream. The fifth stream advantageously allows for keeping a cell concentration constant in the reactor and hence, enables to operate the reactor in steady-state conditions. It will be convenient to note that the fifth stream need not, and typically will not be withdrawn through a membrane, since the purpose here is to remove part of the larger sized compounds (e.g., cells) from the reactor medium.

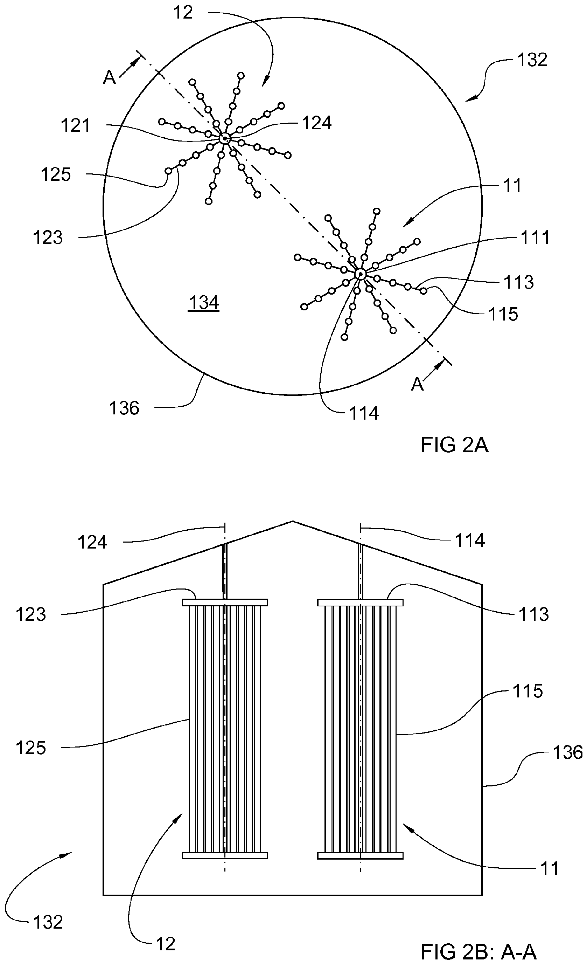

According to an aspect, and referring to Figs. 2A-B, the membranes of units 11 and 12 are moveably arranged within the reactor 13. In particular, the membranes of units 11 and 12 are arranged to move relative to the reactor 13, e.g. vessel 136 of the second reactor 132. Each of the first and second membrane units 11, 12 may comprise a support frame 113, 123 respectively which is pivotally arranged relative to vessel 136. Support frames 113, 123 may be configured to turn on pivot axes 114, 124 fixed to the reactor vessel, such as through a rotating or oscillating motion.

Respective membranes 115, 125 of the first and second membrane units 11, 12 are mounted to the respective support frames 113, 123. The membranes are advantageously fixedly attached to the respective support frames. Each of the membranes 115, 125 comprises a first, outer surface in (direct) contact with, and advantageously immersed in the reactor medium 132, and an opposite, second (inner) surface which communicates with the respective outlet duct 111, 121 through an appropriate manifold, which may be integrated in frame 113, 123. The membrane 115, 125 advantageously provides for transport of compounds from the outer surface to the inner surface, while other compounds may be retained or rejected at the outer surface. The membranes 115, 125 are advantageously hollow fibre membranes or tubular membranes. even though planar membranes, so called flat sheet membranes, which may be arranged flat (e.g. to act like impellers) or may be spirally wound, can be used as well. The support frames 113, 123 and the disposition of the membranes 115, 125 thereon may have any desired configuration. Specific, non-limiting examples of such a configuration are support frames formed as radial brackets (as shown in Fig. 2A), concentric rings, brackets winding spirally about the pivot axis, etc.

When the support frames 113, 123 with the membranes 115, 125 are turned on axes 114, 124, the reactor medium is advantageously mixed. The support frames and membranes therefore may be used in replacement of, or additional to impellers in the reactor. This minimizes concentration and temperature gradients in the vessel, minimizes concentration polarization effects on the surface of the membranes and/or reduces fouling of the membranes 115, 125. The motion of support frames 113, 123 may be continuous or intermittent. Specific examples of this motion are a continuous rotation on axes 114, 124, and an oscillating motion, e.g. back and forth on axes 114, 124, advantageously over an angle ranging between 120° and 240°, e.g. 180°. An actuator, such as an electric motor, possibly arranged outside the reactor vessel 136, may be coupled to the axis 114 and/or 124 to provide the pivoting motion. It will be convenient to note that even though both membrane units 11 and 12 are drawn in a same reactor vessel in Figs. 2A-B, this is no requirement. The first and second membrane units may each be disposed in any one of the available reactors 131, 132, 133 containing a reactor medium. By way of example, the support frame 113 and membranes 115 of the first membrane unit 11 may be arranged in the third reactor 133, while the support frame 123 and membranes 125 may be arranged in the second reactor 132.

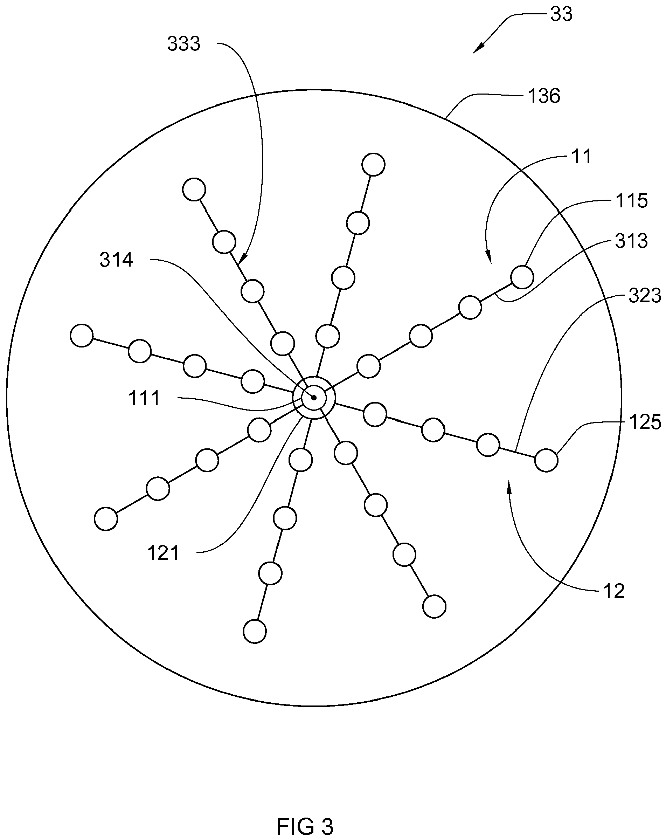

Fig. 3 shows an alternative configuration of a reactor 33. Reactor 33 differs from reactor 13 in that the first and second membrane units 11, 12 are mounted on a common support frame 333, which is pivotally arranged on axis 314 fixed with respect to reactor vessel 136. Support frame 333 advantageously comprises first brackets 313 onto which the membranes 115 relating to the first membrane unit 11 are mounted, and second brackets 323 onto which the membranes 125 relating to the second membrane unit 12 are mounted. The first and second brackets may be disposed as desired on pivot axis 314. By way of example, the first and second brackets may be disposed alternatingly about axis 314. Each one bracket 313, 323 advantageously comprises a collector manifold for collecting the respective first and second streams 15, 16 from the membranes, and communicating with the corresponding outlet duct 111, 121.

As with reactor 13, the support frame 333 of reactor 33 may be configured to turn on axis 314 with any desired motion, e.g. (continuous) rotation or oscillation. The shape of the support frame is not particularly limited, and any suitable shape and/or disposition of the brackets 313, 323 may be used. Possible configurations for the support frame and which can be used in the reactors described herein, are described in US 2009/0034358 to Brod et al. and US 8328167 to Kauling et al. It should be noted that in the above documents, the rotating membrane units are configured for gassing of liquids, whereas in the present description the rotating membrane units are configured for withdrawing two distinct streams from the reactor medium.

The support frames 113, 123 of the first and second membrane units 11, 12, or the support frame 333 placed inside the reactor vessel 136, are advantageously moved relative to the reactor, e.g. by imparting a pivoting motion on axis 114, 124 or 314 oscillating back and forth over a predetermined angle, e.g. 180° or smaller.

The membranes 115, 125 mounted on the respective support frame are in contact with the reactor medium. As the membranes move integrally with the support frame, a relative motion between the reactor medium and the membranes is sustained. In the examples of Figs. 2A-B and 3, the (hollow fibre) membranes have longitudinal axes oriented parallel to the pivot axes 114, 124, 314, and therefore are oriented perpendicular to the plane of motion. These configurations may be effective in maintaining a sufficient level of cross flow of the reactor medium over the external surface of the membranes.

In the first membrane unit 11, a suitable pressure difference across the membranes 115 (the transmembrane pressure) is advantageously applied, e.g. via pump 112. This will withdraw a first stream 15 from the reactor medium which is collected in permeate channels (e.g., the internal lumens of membranes 115) of the membrane unit 11 and further evacuated via a collector manifold to the outlet duct 111. Through suitable selection of the type of membrane 115 (e.g., microfiltration, ultrafiltration, etc.), specific compounds may be selectively recovered from the reactor medium, e.g. on the basis of molecule size. Membranes 115 may be selected to block passage of the microorganisms present in the reactor, which will be retained in the reactor. As a result, cell concentration in the reactor(s) is increased, which will increase consumption of feedstock and therefore increase production rate of the organic solvent(s). By way of example, even though Clostridial strains are known to consume C5 carbohydrates, consumption rates are observed to be low in comparison to C6 consumption rates. This hampers solvent productivities and leads to bulky fermentors. A filtration membrane unit allows cell retention and by consequence will lead to improved xylose conversion.

It will be convenient to note that filtration membranes are just one exemplary type of membranes that can be used in the apparatuses and processes described herein, e.g. in the first membrane unit 11. Other possible membranes may be configured to entrain a selective mass transfer across the membranes via mechanisms other than differential pressure and/or characteristic pore size, such as though not limited to: characteristic attraction of specific charge types (e.g. an ion exchange membrane), selective sorption, or solution diffusion characteristics.

As shown in Fig. 1A and Fig. 1B, the first stream 15 may be introduced in another reactor stage, e.g. a downstream stage, such as the third reactor 133, or may be further processed to separate the individual compounds by an appropriate downstream processing, e.g. by distillation.

The pervaporation or contactor membranes 125 of the second membrane unit 12, when applying a suitable partial vacuum or reduced pressure at the internal membrane surface (permeate side), e.g. via pump 122, will withdraw a second permeate from the reactor medium which is collected in permeate channels (e.g., the internal lumens of membranes 125) of the membrane unit 12 and further evacuated via a collector manifold to the outlet duct 121 as the second stream 16. In case the second membrane unit 12 is arranged as a pervaporation unit, the second stream is typically collected at the internal face of membranes 125 as a vapour. The second stream 16 may comprise more than one compound. The second stream advantageously comprises volatile organic compounds, such as the organic solvent(s), and may comprise water. Withdrawing the organic solvent(s) from the reactor medium alleviates product toxicity and improves the water balance by increasing the carbohydrate utilization. titerThe second stream 16 may be condensed to a liquid phase, e.g. through condenser 161, and may be separated into its separate compounds by an appropriate downstream processing.

Pervaporation membranes 125 may be porous or nonporous membranes allowing a selective mass transfer driven by a partial vapour pressure gradient between feed and permeate side. In nonporous membranes, a sorption-diffusion-desorption process is sustained through the membrane. Membranes for pervaporation are typically thin film composite membranes, comprising a separation layer defining the membrane characteristics provided on a porous support. Materials for the separation layer useful for pervaporation purposes may be made from a polymer material, a zeolite, a ceramic material or combinations thereof (e.g., polymer membranes with zeolite or ceramic filler particles). The membranes may be either hydrophilic, or organophilic (hydrophobic), and organophilic membranes are preferred for applications described herein. Specific examples of useful organophilic membranes are made of polydimethylsiloxane (PDMS), Poly(octyl methyl siloxane) (POMS), or ZSM-5 (MFI) zeolite.

Membranes used in membrane contactors may be porous or nonporous membranes. In non-porous membranes, a sorption-diffusion-desorption process is sustained through the membrane. Also here, membranes are typically thin film composite membranes. Materials for the separation layer useful for membrane contactor purposes may be made from a polymer material, a zeolite, a ceramic material or combinations thereof.

One advantage of the apparatuses and processes described herein is that they are inherently safer because no associated (external) loops with high cross flows are involved. There is no or less external piping which may be prone to leakage and/or which may expose maintenance operators to (potentially) toxic products. There is also no need for heat tracing in associated loops to keep the temperature constant in the loops. A further advantage is that it has a small footprint, taking much less space in comparison to prior art designs. Yet an additional advantage is that due to the integration of the membrane units 11 and 12 within the reactor, required pumping energy is greatly reduced.

It will be convenient to note that additional membrane units may be integrated in the reactor, similarly to the principles for the first and second membrane units 11, 12 described herein. Referring to Figs. 1A and 1B, a fifth membrane unit 20 may be provided in the downstream reactor stage 133. The fifth membrane unit may be similar as the first membrane unit 11, e.g. arranged as a filtration unit comprising microfiltration or ultrafiltration membranes, or alternatively may be similar to the second membrane unit 12.

Furthermore, the third membrane unit 18 may be provided in the reactor, either on a separate support frame, or integrated on the support frame of either or both the first and the second membrane units. The support frame of the membranes of the third unit may be pivotally arranged relative to the reactor, or alternatively may be fixed within the reactor. Yet further membrane units may be provided, e.g. configured for dosing liquid or gaseous compounds to the reactor medium, through suitable porous or nonporous membranes.

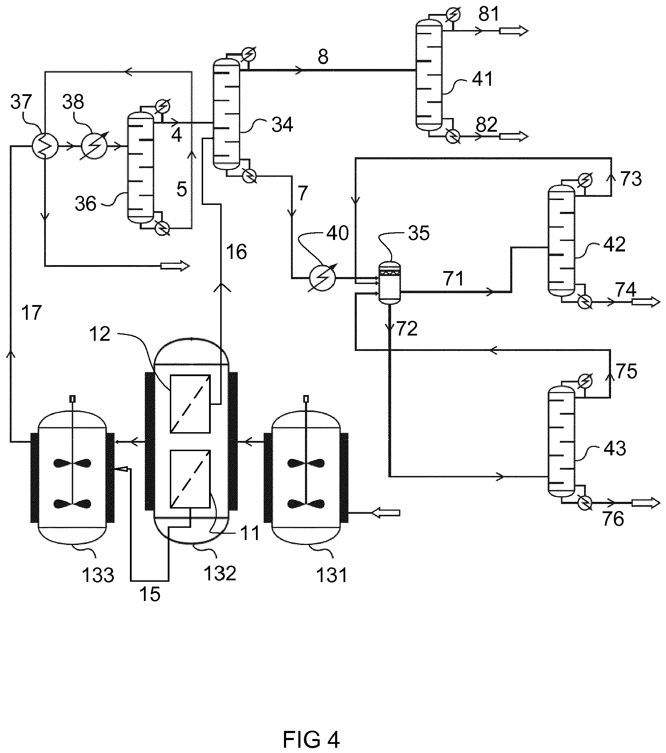

The reactor configurations described above in relation to Figs. 1-3 are advantageously used for processes involving forming one or a mixture of organic solvents, as described herein, such as ABE. Conventional downstream processing may be applied to the second stream 16 and/or the third stream 17 to obtain the organic solvent at desired purity grades. One specific example of a possible downstream process for the second stream 16 and the third stream 17 will now be described in relation to Fig. 4, in particular for the case in which the second stream 16 originates from a membrane unit 12 being a pervaporation unit. The second stream 16 is supplied as feed to a first distillation column 34, possibly as a condensate following passing through a condenser 161 (see Fig. 1). The second stream 16 is distilled in column 34 to produce an overhead stream 8, enriched in a first range of solvents, and a bottoms stream 7 which may be liquid, depleted in the first range of solvents. Distillation column 34 is advantageously a multistage distillation column, such as comprising a number of theoretical stages ranging between 6 and 35.

The third stream 17, or at least a portion thereof may also be fed to the first distillation column 34. To this end, third stream 17 can first be sent to a stripper, e.g. a steam stripper 36. In the context of the present description, a steam stripper refers to a beer stripper or steam distillation apparatus, known by those skilled in the art. The third stream 17 may be centrifuged for cell/particle removal prior to sending the (cell/particle-free) effluent to the stripper. In addition, or alternatively, third stream 17 may be heated, e.g. by passing through heat exchangers 37, 38, prior to being fed to the stripper. By way of example, heat exchanger 37 may be configured to heat stream 17 to a temperature comprised between 75°C to 85°C, after which the stream 17 may be further heated by heat exchanger 38, e.g. to a temperature comprised between 90°C to 95°C, and afterwards sent to the steam stripper 36. Steam stripper 36 produces a top (overhead) stream 4 which is fed to the first distillation column 34, where it is distilled in conjunction with the second stream 16 to produce bottoms stream 7 and overhead stream 8.

Bottoms stream 7, exiting the first distillation column 34 may comprise two phases, a solvent rich phase and an aqueous phase. In one specific example, the solvent rich phase comprises n-butanol and the aqueous phase comprises water. These two phases are subsequently separated in a suitable separator. By way of example, bottoms stream 7 is fed to a decanter 35, where stream 7 is separated in the solvent rich phase 71, and the aqueous phase 72. In ABE fermentative processes, the solvent rich phase 71 advantageously is mainly formed of n-butanol, e.g. at least 51% by volume. The aqueous phase 72 may comprise a lesser amount of n-butanol, e.g. less than 15% by volume, such as between 3% to 15% by volume n-butanol.

A heat exchanger 40 may be arranged between the first distillation column 34 and the decanter 35 to adjust a temperature of the bottoms stream 7 to a temperature suitable for decantation. A suitable temperature for decantation depends on the products, advantageously solvents, that need to be separated and will be apparent for those skilled in the art. By way of example, the temperature of stream 7 upon feeding to decanter 35 ranges between 35°C and 45°C.

The solvent rich phase 71 is advantageously further purified, e.g. by distillation. To this end, phase 71 may be fed to a second distillation column 42, communicating with a first outlet of the decanter 35. Second distillation column 42 generates a bottoms stream 74 forming a first solvent, advantageously a substantially pure first solvent. In ABE fermentations, stream 74 advantageously comprises, or consists of (substantially pure) n-butanol. Advantageously, stream 74 consists of n-butanol having a purity comprised between 99.0% (w/w) and 99.9% (w/w), advantageously between 99.5% (w/w) and 99.9% (w/w), advantageously between 99.7% (w/w) and 99.9% (w/w), advantageously a purity of 99.8% (w/w).

The produced (substantially pure) n-butanol in aspects of the invention can be used as an intermediate in chemical industry. For example, the produced n-butanol can be used as a solvent, as a feedstock chemical in the plastics industry, as an ingredient in formulated products such as cosmetics, as a food grade extractant in the food and flavour industry, as a fuel, or as a fuel additive. The overhead stream 73 exiting second distillation column 42 can be recycled back to the decanter 35 for decantation.

The aqueous phase 72 exiting the decanter 35 as a bottoms stream is advantageously further purified, e.g. by distillation. To this end, phase 72 can be sent to a third distillation column 43, communicating with a second outlet of the decanter 35. The third distillation column 43 generates a bottoms stream 76 forming a substantially pure aqueous phase, depleted from organic solvent. Advantageously, stream 76 comprises only trace amounts of (organic) solvents, advantageously stream 76 comprises mainly water depleted from n-butanol. The third distillation column 43 generates an overhead stream 76 which can be recycled back to the decanter 35.

In an alternative embodiment, the aqueous phase 72 formed by decantation in the decanter 35 as a bottoms stream, can be sent to an extraction unit (not shown). In such an extraction unit, n-butanol is extracted out of water using a (bio)diesel or another fuel as extractant. This results in the production of a n-butanol enriched (bio)fuel, which can be further used as such.

Overhead stream 8 of the first distillation column 34 may comprise amounts of organic solvents. In ABE fermentation, for example, stream 8 may comprise a mixture of mainly acetone and ethanol. The obtained mixture comprising acetone and ethanol can directly be used in further chemical reactions. Alternatively, overhead stream 8 may be further purified/separated, e.g. by distillation to obtain a stream of (substantially pure) acetone and a separate stream of an azeotropic mixture of ethanol. To this end, overhead stream 8 can be sent to a fourth distillation column 41, communicating with a second outlet of the first distillation column 34. The fourth distillation column 41 generates an overhead stream 81 comprising, or consisting of a (substantially pure) second solvent and a bottoms stream 82 comprising a third solvent (and being depleted in the second solvent). In ABE fermentation, stream 81 advantageously comprises or substantially consists of acetone. The obtained acetone in stream 81 may have a purity comprised between 98.0% (w/w) and 99.9% (w/w), advantageously between 98.5% (w/w) and 99.5% (w/w), advantageously between 99.0% (w/w) and 99.5% (w/w). Stream 82 may be rich in ethanol. By way of example, stream 82 may be an azeotropic mixture (or azeotropic solution) comprising between 85% and 90% by volume ethanol and between 10% and 15% by volume water, advantageously between 86% and 88% by volume ethanol and between 12% and 14% by volume water, advantageously 87% by volume ethanol and 13% by volume water.

## Claims

1. Method of producing an organic solvent, comprising:
adding a feed (14) to a medium (138) comprised in a reactor (13, 33),
converting the feed in the reactor to form the organic solvent,
separating a first stream (15) from the medium through a first membrane (115),
separating a second stream (16) from the medium through a second membrane (125), the second stream comprising the organic solvent,
wherein the first stream and the second stream are withdrawn separately from the reactor,
wherein the first membrane and the second membrane are moved relative to the reactor during the steps of separating the first stream and separating the second stream.

2. Method of claim 1, wherein the steps of separating the first stream and of separating the second stream are performed in parallel.

3. Method of claim 1 or 2, wherein the first membrane and the second membrane are immersed in the medium.

4. Method of any one of the preceding claims, wherein the first membrane and the second membrane are mounted on respective support frames (113, 123, 313, 323) arranged in the reactor, the method comprising turning the support frames relative to the reactor.

5. Method of claim 4, wherein turning the support frames comprises rotating or oscillating the support frames about an axis (114, 124, 314) fixed to the reactor.

6. Method of any one of the preceding claims, wherein separating the first stream comprises filtrating the medium through the first membrane to obtain a filtrate being the first stream (15).

7. Method of claim 6, wherein the first membrane is a microfiltration, ultrafiltration or nanofiltration membrane.

8. Method of any one of the preceding claims, wherein the second stream is separated through the second membrane via pervaporation or liquid-liquid extraction.

9. Method of claim 8, wherein the second stream (16) is separated through the second membrane by pervaporation, the second stream comprising a gaseous compound, in particular carbon dioxide.

10. Method of any one of the preceding claims, wherein the feed is converted through a fermentation process.

11. Method of claim 10, wherein the medium comprises a microorganism.

12. Method of claim 11, wherein the microorganism is of the Clostridium genus.

13. Method of claim 11 or 12, wherein the first membrane is a porous membrane with a characteristic pore size retaining the microorganism in the medium.

14. Method of any one of the preceding claims, wherein the organic solvent is butanol or acetone.

15. Method of any one of the preceding claims, wherein the feed comprises a carbohydrate feedstock.
